Europäisches Patentamt

European Patent Office

Office européen des brevets

(19)

(11) Publication number: **0 022 592**
**B1**

(12) **EUROPEAN PATENT SPECIFICATION**

(45) Date of publication of patent specification: **28.09.83**

(51) Int. Cl.³: **A 01 M  1/20, A 01 M  1/24**

(21) Application number: **80200621.3**

(22) Date of filing: **30.06.80**

(54) Biocide dispenser and method of applying biocide to a surface.

(30) Priority: **16.07.79 GB  7924654**

(43) Date of publication of application:
**21.01.81 Bulletin 81/3**

(45) Publication of the grant of the patent:
**28.09.83 Bulletin 83/39**

(84) Designated Contracting States:
**AT BE CH DE FR GB IT LI NL SE**

(56) References cited:
**AT - B - 176 302**
**AT - B - 320 337**
**CH - A - 58 698**
**DE - A - 2 810 468**
**DE - C - 411 831**
**FR - A - 1 415 759**
**FR - A - 1 537 911**
**GB - A - 910 563**
**US - A - 1 960 387**
**US - A - 2 456 324**

(73) Proprietor: **SHELL INTERNATIONALE RESEARCH MAATSCHAPPIJ B.V.**
**Carel van Bylandtlaan 30**
**NL-2596 HR  Den Haag (NL)**

(72) Inventor: **Tester, Stuart Alan**
**2 The Laurels**
**Fleet Hampshire (GB)**
Inventor: **Twydell, Roland Stephen**
**19 Lansdowne Road**
**Sittingbourne Kent (GB)**

(74) Representative: **Rogers, Roy Charles et al,**
**4 York Road**
**London SE1 7NA (GB)**

Courier Press, Leamington Spa, England

# 0 022 592

### Biocide dispenser and method of applying biocide to a surface

This invention relates to a dispenser for the application of insecticides and/or fungicides onto a surface.

It is known to apply insecticide and fungicide formulations to the surfaces of plants and structures and objects, for example, fencing, by applicational techniques utilising spraying devices, brushes and applicators of the dauber type. All such techniques apply relatively large amounts of the formulation to the surface to be treated.

The present invention is predicated on the discovery that the application of an insecticide and/or fungicide formulation in a thin, relatively narrow line to the surface of a structure or object, typically in a domestic situation, surprisingly can provide useful and adequate protection against insects and/or fungal growth.

Accordingly, the present invention provides a dispenser for use in providing insecticidal and/or fungicidal protection on a surface of a structure or object, which comprises a reservoir containing a fluid or semi-fluid insecticide and/or fungicide formulation, said reservoir having an outlet orifice which is closed by a plug of such a size and nature that when the dispenser is drawn over said surface with said plug in frictional contact therewith it releases said formulation onto said surface to form a single thin, relatively narrow line thereof on said surface.

The present invention also provides a method of providing insecticidal and/or fungicidal protection on a surface of a structure or object, which comprises drawing a dispenser as just defined over said surface with its plug in frictional contact therewith so as to form a thin, relatively narrow line of insecticide and/or fungicide formulation thereon.

The dispenser reservoir may be of any convenient shape or material, such as a bottle or tube of glass, metal or plastic, and conveniently it contains a cellular filling which absorbs the insecticide and/or fungicide formulation (hereinafter referred to for convenience as a biocide formulation), and prevents its uncontrolled movement within the reservoir. Suitable filling materials include cellulose acetate, cellulose and polyether fibres. The plug may be such as to permit controlled emission of the biocide on the application of external pressure, and may be realized in a variety of embodiments. In one embodiment, the plug may be formed from any porous material which permits the restricted, controlled flow of biocide formulation out of the reservoir and onto the surface to be coated, such as natural or synthetic felt, porous elastomer, etc. Alternatively, the plug may comprise a spring-loaded valve which opens under pressure to permit flow of the biocide formulation, or a movable ball as in "roll-on" deodorant dispensers. In practical application, the dispenser would normally be provided with a removable cap covering the outlet plug so as to prevent release of biocide prematurely in inappropriate locations. A particularly convenient and preferred form of the dispenser can be simply envisaged as analogous to a felt marker pen in which the ink fluid is replaced by the biocide formulation. Thus, in this preferred form, the dispenser comprises an elongated, hollow, substantially cylindrical container holding a fluid or semifluid formulation of biocide, which is preferably absorbed within a cellular filling, and provided at one end with a fibrous plug, suitably felt, which permits capillary flow of biocide formulation out from the container.

The biocide formulation should, of course, be designed so as to maintain a stable vehicle for the biocide, and to permit adequate flow through the plug without leaving residues which hinder the continued efficient operation of the plug. The concentration of biocide in the formulation is naturally dependent on the intrinsic activity of the compound, but in general it is found that amounts between 1% and 25%, suitably around 5%, by weight are satisfactory. The precise composition of the formulation will naturally be dependent on the physical and chemical properties of the biocide itself, but in general it is found convenient to use a solution in an organic solvent such as an alkanol, e.g. propanol, an aromatic hydrocarbon solvent, e.g. "Shellsol" A, or a glycol ether, e.g. "Dioxitol", optionally in conjunction with an oil (such as "Risella" D15; "Shellsol", "Dioxitol" and "Risella" are Registered Trade Marks) to improve flow and also reduce volatility of the solvent system. The use of alcohol solvents with aluminium containers is inadvisable since chemical reaction frequently leads to pinholing. The proportions of these vehicles naturally depend on the properties required, but good results have been obtained using a formulation comprising 5% insecticide, from 45—75% "Shellsol" A, and from 20—50% "Risella" D15, preferably 75% "Shellsol" A and 20% "Risella" D15.

The nature of the biocide is naturally dependent on the specific type of biocidal activity required, and a particularly useful application of this dispenser is for insecticide application. It is observed that flies are frequently found at or near windows, so that an effective control of flies can easily be obtained by using the Applicants' dispenser to apply insecticide to window sills, or to the window itself. The insecticide used may be either a contact insecticide, such as a pyrethroid, e.g. permethrin, cypermethrin or fenvalerate, an insecticidal carbamate or an organo-phosphate, or a volatile insecticide, such as dichlorvos. It will be appreciated that a contact insecticide can also be very effective for control of crawling insects (e.g. cockroaches and ants) if the dispenser is used to apply the insecticide to those surfaces which are known to be regularly frequented by such insects. However, the practical application of this dispenser is not limited solely to insecticides. It is often found that unsightly fungus can

**0 022 592**

develop on areas of wood or grouting exposed to dampness, as in the edge of bathroom fittings, or window frames and putty exposed to condensation moisture, and such fungus can now be readily controlled by using the Applicants' dispenser to apply a suitable fungicide formulation to the areas affected. The fungicide could be effective either by direct contact or by vapour action, suitable materials including Triforine, Proxel, Vantoc (all Trademarks) and 2,5-dichloro-atroponitrile. A dual control of insects and fungus can be obtained by using a biocide formulation containing both an insecticide and a fungicide. Accordingly, the invention includes also a method for the control of insects and/or fungi, which comprises applying a biocide to a surface subjected to contact by insects and/or infestation by fungi by means of the dispenser of this invention. Although alternative methods of applying a biocide to a surface are available, for example by means of a biocide-containing semi-solid formulation projecting from a holder (analogous to lipstick) or from a vessel containing a liquid biocide formulation, suitably by means of a brush attached to the vessel cap (as with vehicle touch-up paints), but the use of the dispenser of this invention affords certain advantages of practicality and convenience. The surface chosen may be any area frequently approached by insects, but best results have been obtained by the application of biocide to the inner surface of the window and/or window frame.

The invention and its practical application are further illustrated in the following Examples.

EXAMPLE 1

A cylindrical tube 125 mm × 19 mm o.d. of polypropylene or aluminium is sealed at one end, and the open end surmounted by a cone 25 mm long which reduces the tube diameter to 7 mm o.d. A nib composed of felt (natural or synthetic) is fitted into the conical end of the tube so that its stem reaches into the main body of the holder, whilst its chisel-shaped nib (6 mm × 2 mm cross section) remains exposed. Within the main reservoir body of the tube is a cellular filling composed of cellulose acetate. When not in use, the conical end and exposed nib are closed by a cap of polypropylene or polyamide. Formulations of biocide to be evaluated were prepared, and a measured dosage of 10 g introduced into the reservoir of the pen by immersion and capillary action over a 3 minute period.

EXAMPLE 2

The practical efficacy of the dispenser pens of Example 1 charged with different insecticides was evaluated against houseflies (*Musca domestica*) in a standard Peet Grady chamber in which the conventional window in one wall is replaced by a glass panel on which insecticide has been applied by the pen. This was effected by drawing a continuous line with the pen horizontally across the glass panel, which typically deposited the formulation in an amount of between 1—25, preferably 10, mg per linear meter of glass. After evaporation of the solvent the resulting insecticide deposit is virtually transparent and invisible, and since the formulation typically contains 5% m/m of insecticide the mass of deposit is about 1 mg for each linear meter of application. The actual rate of evaporation and precise appearance of the residual line is naturally dependent upon the solvents used and the insecticide itself, as is also the duration of the insecticidal effect produced, but in general the evaporation will be complete within a few days and the activity will remain for several weeks.

The specific formulations evaluated were solutions of the following composition (percentages being m/m):—

TABLE 1

| Ref. No. | Insecticide | Solvent | | | | |
|---|---|---|---|---|---|---|
| 1 | Permethrin | 5% | "Risella" D15 | 50%; | "Shellsol" A | 45% |
| 2 | " | " | "Dioxitol" | 50%; | " | " |
| 3 | Fenvalerate | " | "Risella" D15 | 50%; | " | " |
| 4 | " | " | "Dioxitol" | 50%; | " | " |
| 5 | Permethrin | " | "Risella" D15 | 30%; | " | 65% |
| 6 | " | " | " | 50%; | " | 45% |
| 7 | " | " | " | 50%; | Isopropanol | " |
| 8 | Cypermethrin | " | " | 50%; | "Shellsol" A | " |
| 9 | " | " | " | 50%; | Isopropanol | " |

3

Permethrin is 3-phenoxybenzyl 2,2-dimethyl-3-(2,2-dichlorovinyl)cyclopropane carboxylate.

Cypermethrin is the alpha-cyano analogue of permethrin, and fenvalerate is alpha-cyano-3-phenoxybenzyl-alpha-isopropyl-4-chlorophenylacetate.

In the case of formulations 1—4, the knock-down was expressed as a percentage measured after varying time intervals, whilst for formulations 5—9 it was expressed as $KD_{50}$, being the time in minutes to achieve knock-down of 50% of the flies, together with the total known-down after 1 hour. The results are shown in the following Table 2.

TABLE 2

| Form | Deposit, mg/m | % Knock-down after | | | $KD_{50}$ (mins) | Total 1 hr KD |
|---|---|---|---|---|---|---|
| | | 15 | 30 | 45 | | |
| 1 | 0.9 | 47 | 75 | 86 | | 95 |
| 2 | 0.8 | 19 | 61 | 81 | | 91 |
| 3 | 0.9 | 39 | 75 | 87 | | 95 |
| 4 | 0.8 | 5 | 31 | 64 | | 81 |
| 5 | 1.0 | | | | 30 | 81 |
| 6 | 1.0 | | | | 33 | 86 |
| 7 | 0.6 | | | | 52 | 61 |
| 8 | 0.9 | | | | 23 | 94 |
| 9 | 0.9 | | | | 20 | 98 |

EXAMPLE 3

An evaluation was also carried out to obtain an indication of the efficacy of the dispenser pen in providing control of cockroaches (*Blatella germanica*). 2 adjacent lines were drawn on a test surface of glass or plywood, and a number of cockroaches exposed to the treated surface for 5 minutes and then transferred to a clean recovery vessel. The results were expressed as $KD_{90}$, i.e. the time in minutes to achieve knock-down of 90% of the exposed individuals, and are set out in Table 3 below.

TABLE 3

| Form | Surface | Age of deposit (hrs) | $KD_{90}$ |
|---|---|---|---|
| 8 | Glass | 0.5 | 20 |
| 9 | Plywood | 0.5 | 20 |
| | | 72 | 45 |
| 7 | " | 0.5 | c.45 |
| | " | 72 | not attained |

In preliminary, qualitative trials, the dispenser pens were also used on doorsteps, shelves, etc. to provide a deposit giving effective control of ants.

EXAMPLE 4

The dispenser pens of Example 1 were charged with a 10% (m/m) solution of dichlorvos in RISELLA EL/SHELLSOL A (20 + 70 by mass). The activity of these samples was determined by bioassay against *Musca domestica* using the Peet Grady chamber. The results are set out in Table 4 and indicate that the volatility of dichlorvos would necessitate daily application of the product to provide acceptable insect control.

TABLE 4

| Age of deposit (hours) | Mass of formulation applied (mg)* | % knockdown after (minutes) | | | | % Mortality |
|---|---|---|---|---|---|---|
| | | 15 | 30 | 45 | 60 | |
| 1 | 12.6 | 30.1 | 35.8 | 54.7 | 73.8 | 70 |
| 3 | 12.4 | 3.5 | 8.8 | 26.3 | 64.9 | 70 |
| 24 | 12.4 | 8.8 | 17.5 | 21.1 | 33.3 | 50 |
| 30 | 12.6 | 0 | 11.6 | 11.6 | 23.3 | 50** |

*Note:*

\* This is equivalent to approximately 1.9 mg dichlorvos per metre of line drawn.
\*\* Assessed on 10 knocked down flies as opposed to normal 20 taken.

EXAMPLE 5

The dispensers were evaluated for their effectiveness in the prevention of fungal growth by the following tests.

The fungicides evaluated were:

(a) quaternary ammonium salt of 1,2-benzithiazolin-3-one.

Supplied as 33% aqueous glycol solution (Proxel AS ICI, Trade Mark), and

(b) benzalkonium chloride solution (Vantoc CL ICI, Trade Mark).

Simple aqueous solutions of both fungicides were used at concentrations of 0.32, 1.0 and 3% (saturated) (m/m) active ingredient for Proxel AS and 0.1, 1.0 and 10% (m/m) active ingredient for Vantoc CL. In addition, an (unstable) 20% (m/m) suspension of Proxel AS was used. Eight grams of each of the these solutions were filled into standard dispensers, viz:

aluminium body; juxtafelt nib;

TBU 3043 (soft) reservoir.

Fungal spore suspensions of *Aspergillus niger* (T346 obtained from Pest Infestation Lab., Slough); *Cladosporium herbarum* (IMI 170 353); or *Aureobasidium pullulans* (Lab. strain, not from culture collection) were obtained by washing from the surfaces of fungal cultures (grown on malt extract agar at 25°C for 10 days). The resulting spore suspension were transferred to sterile Universal bottles and agitated using a Whirly-mixer. The suspensions were then filtered through a layer of sterile glass wool to provide homogeneous spore suspensions. The spore suspensions (1 ml) were then diluted in 20 ml of malt extract broth to provide spore concentrations of $10^7$ ml$^{-1}$. These suspensions were applied to wood surfaces which have been prepared as follows.

Sections of untreated softwood (75 mm × 40 mm × 7 mm) were either left unpainted, or were painted with an unpreserved PVA emulsion paint supplied by ICI, and were steeped in malt extract broth (Oxoid, Trade Mark) for 1 hour at room temperature. They were then placed in glass petri-dishes and sterilized by autoclaving at 121°C for 20 minutes.

Two procedures were followed:

(a) Application of the spore suspensions to the wood surface (approximately 0.15 ml spread using a glass spreader), allowing to stand for 4 hours, and then applying the fungicide from the dispenser pen over the surface (using hand pressure at an approximate speed of 3 cm sec.$^{-1}$).

(b) Applying the fungicide over the surface as in (a) and then applying the spore suspensions.

In all cases the blocks were incubated, after treatment, in glass petri-dishes at 25°C for 7 days. They were then observed for fungal growth on the surfaces. If fungal inhibition had occurred a clear region of "no growth" would be seen in the region of application of the fungicides.

The results from method (a) are shown in Table 5 and from method (b) in Tables 6 and 7. The concentrations given are those in the formulations and it is unlikely that these concentrations were achieved on the wood surfaces.

# 0 022 592

TABLE 5
Effects of fungicides applied from dispenser on
unpainted wood (Procedure (a))

| Fungus | Minimum concentration in dispenser that inhibits on wood | |
|---|---|---|
| | Proxel AS | Vantoc CL |
| Aureobasidium pullulans | 20% | 0.1% |
| Aspergillus niger | saturated solution | 1.0% |
| Cladosporium herbarum | 1% | 0.1% |

TABLE 6
Effects of fungicides applied from dispenser on
unpainted wood (Procedure (b))

| Fungus | Minimum concentration in dispenser that inhibits on wood | |
|---|---|---|
| | Proxel AS | Vantoc CL |
| Aureobasidium pullulans | saturated solution | 1% |
| Aspergillus niger | no effect | 10% |
| Cladosporium herbarum | 0.32% | 1% |

TABLE 7
Effects of fungicides applied from dispenser on
painted wood (Procedure (b))

| Fungus | Minimum concentration in dispenser that inhibits on wood | |
|---|---|---|
| | Proxel AS | Vantoc CL |
| Aureobasidium pullulans | 20% | 10% |
| Aspergillus niger | no effect | 1% |
| Cladosporium herbarum | saturated solution | 1% |

## Claims

1. A dispenser for use in providing insecticidal and/or fungicidal protection on a surface of a structure or object, which comprises a reservoir containing a fluid or semi-fluid insecticide and/or fungicide formulation, said reservoir having an outlet orifice which is closed by a plug of such a size and nature that when the dispenser is drawn over said surface with said plug in frictional contact therewith it releases said formulation onto said surface to form a single thin, relatively narrow line thereof on said surface.

2. A dispenser as claimed in claim 1, wherein said plug is composed of a porous material, for example, felt.

3. A dispenser as claimed in claim 1, wherein said plug comprises a spring-loaded valve or movable ball.

6

**0 022 592**

4. A method of providing insecticidal and/or fungicidal protection on a surface of a structure or object, which comprises drawing a dispenser as claimed in any one of claims 1 to 3 over said surface with its plug in frictional contact therewith so as to form a thin, relatively narrow line of insecticide and/or fungicide formulation thereon.

**Revendications**

1. Un distributeur utilisable pour fournir une protection insecticide et/ou fongicide sur une surface d'une structure ou d'un objet, qui comprend un réservoir contenant une composition insecticide et/ou fongicide fluide ou semi-fluide, ce réservoir ayant un orifice de sortie qui est fermé par un bouchon ayant des dimensions et une nature telles que quand on tire le distributeur sur la surface avec le bouchon en contact frottant avec elle, il libère la composition sur la surface de manière à former une seule ligne fine, relativement étroite de la composition sur la surface.

2. Un distributeur selon la revendication 1, dans lequel le bouchon est composé d'une matière poreuse, par exemple de feutre.

3. Un distributeur selon la revendication 1, dans lequel le bouchon comprend une valve poussée par un ressort ou une bille mobile.

4. Un procédé pour assurer une protection insecticide et/ou fongicide à une surface d'une structure ou d'un objet, selon lequel on déplace un distributeur tel que revendiqué dans l'une quelconque des revendications 1 à 3 sur la surface avec son bouchon en contact frottant avec elle de manière à former une ligne fine, relativement étroite, de composition insecticide et/ou fongicide sur la surface.

**Patentansprüche**

1. Spender zur Schaffung eines insektiziden und/oder fungiziden Schutzes auf einer Oberfläche einer Struktur oder eines Gegenstandes, umfassend einen Behälter, der eine flüssige oder halbflüssige Insektizid- und/ober Fungizid-zubereitung enthält, wobei dieser Behälter eine Austrittsöffnung aufweist, die mit einem Stopfen von solcher Größe und Art verschlossen ist, daß dann, wenn der Spender, während sich der Stopfen in Reibungskontakt mit der Oberfläche befindet, über diese Oberfläche gezogen wird, der Behälter jene Zubereitung auf die Oberfläche unter Bildung eines einzigen, dünnen, relativ schmalen Striches aus der Zubereitung abgibt.

2. Spender nach Anspruch 1, wobei der Stopfen aus einem porösen Material, beispielsweise Filz, besteht.

3. Spender nach Anspruch 1, wobei der Stopfen ein federbelastetes Ventil oder eine bewegliche Kugel umfaßt.

4. Verfahren zur Schaffung eines insektiziden und/oder fungiziden Schutzes auf einer Oberfläche einer Struktur oder eines Gegenstandes, bei welchem ein Spender nach einem der Ansprüche 1 bis 3 über diese Oberfläche gezogen wird, wobei sich der Stopfen des Spenders in Reibungskontakt mit der Oberfläche befindet, um auf derselben einen dünnen, relativ schmalen Strich aus Insektizid- und/oder Fungizidzubereitung zu bilden.